# EUROPEAN PATENT SPECIFICATION

(11) **EP 2 952 897 B1**
(45) Date of publication and mention of the grant of the patent: **22.03.2017**
(21) Application number: 15172303.8
(22) Date of filing: 08.04.2009
(51) Int. Cl.: G01N 33/558, G01N 33/58, B82Y 5/00

(54) **SENSITIVE IMMUNOASSAYS USING COATED NANOPARTICLES**
EMPFINDLICHE IMMUNTESTS MIT BESCHICHTETEN NANOPARTIKELN
IMMUNOESSAIS SENSIBLES À L'AIDE DE NANOPARTICULES REVÊTUES

(30) Priority: 09.04.2008 US 71035 P
(43) Date of publication of application: 09.12.2015
(62) Divisional of application: 09730869.6
(73) Proprietor: Becton, Dickinson and Company, Franklin Lakes, NJ 07417-1880 (US)
(72) Inventor: WEIDEMAIER, Kristin, Raleigh, NC North Carolina 27613 (US); SANDMANN, Christian, Raleich, NC North Carolina 27617 (US); FULCHER, Robert A., Hillsborough, NC North Carolina 27278 (US); KURODA, Melody, Durham, NC North Carolina 27703 (US); ALLPHIN, Lori Pederson, Wake Forest, NC North Carolina 27612 (US); CURRY, Adam C., Raleigh, NC North Carolina 27612 (US)
(74) Representative: dompatent von Kreisler Selting Werner - Partnerschaft von Patent- und Rechtsanwälten mbB

(56) References cited:
- WO-A1-00/31538
- WO-A2-01/18235
- WO-A2-2007/090058
- US-A- 6 136 610
- US-A1- 2005 130 207
- KAMATA ET AL: "Synthesis and Characterization of Monodispersed Core-Shell Spherical Colloids with Movable Cores", JOURNAL OF THE AMERICAN CHEMICAL SOCIETY, AMERICAN CHEMICAL SOCIETY, US, vol. 125, no. 9, 1 March 2003 (2003-03-01) , pages 2384-2385, XP002299225, ISSN: 0002-7863, DOI: 10.1021/JA0292849
- DOERING W E ET AL: "SERS as a foundation for nanoscale, optically detected biological labels", ADVANCED MATERIALS 20071019 WILEY-VCH VERLAG DE, vol. 19, no. 20, 19 October 2007 (2007-10-19), pages 3100-3108, XP002538667,
- WANG S. ET AL.: "Development of colloidal gold-based flow-through and lateral-flow immunoassays for the rapid detection of the insecticide carbaryl", ANALYTICA CHIMICA ACTA, vol. 546, 17 June 2005 (2005-06-17), pages 161-166, XP002538668,

## Description

### FIELD

Coated nanoparticles comprising a core surrounded by a shell that increases the reflectance of the nanoparticle, wherein the coated nanoparticle need not, but optionally can, include a Raman-active molecule, are provided. The coated nanoparticles disclosed herein are useful in test devices and methods for quantitative and/or qualitative determination of the presence or absence of an analyte in a liquid sample.

### BACKGROUND

Immunoassay technology provides a simple and relatively rapid means for determining the presence or absence of analytes in biological samples. The information provided from immunoassay diagnostic tests are often critical to patient care. Assays are typically performed to detect qualitatively or quantitatively the presence of particular analytes, for example, antibodies that are present when a human subject has a particular disease or condition. Immunoassays practiced in the art are numerous, and include assays for diseases, such as infections caused by bacteria or viruses, or conditions, such as pregnancy.

Various types of immunoassays are known in the art. One type of immunoassay procedure is the lateral flow immunoassay. Lateral flow assays utilize a solid support, such as nitrocellulose, plastic, or glass, for performing analyte detection. Instead of drawing the sample through the support perpendicularly, as in the case of a "flow-through" assay, the sample is permitted to flow laterally along the support by capillary and other forces from an application zone to a reaction zone on the surface. In a lateral flow assay, capture antibodies are striped onto the solid support. Detection antibodies are conjugated to a detection molecule, which provides a signal that is detectible. A liquid sample is placed in contact with the detection antibodies, and the sample/detection antibody mixture is allowed to flow along the solid support. If the analyte is present, a "sandwich" complex is formed at the location on the solid support where the capture antibodies have been striped. The signal from the detection molecule localized at the capture line is then detected, either visually or with an instrument. A lateral flow assay can be configured to detect proteins, nucleic acids, metabolites, cells, small molecules, or other analytes of interest.

Another immunoassay format is a flow-through immunoassay. A flow-through immunoassay generally uses a porous material with a reagent-containing matrix layered thereon or incorporated therein. Test sample is applied to and flows through the porous material, and analyte in the sample reacts with the reagent(s) to produce a detectable signal on the porous material. These devices are generally encased in a plastic housing or casing with calibrations to aid in the detection of the particular analyte.

Many examples of different types of detection molecules useful in lateral flow immunoassays are known in the art, such as fluorophores, gold colloids, labeled latex particles, and nanoparticles such as a quantum dot or a surface enhanced Raman scattering ("SERS") nanoparticle. For example, U. S. Patent No. 5,591,645 describes the use of visible "tracer" molecules, such as colloidal gold, that can be seen without the use of instrumentation. In addition, U.S. Patent No. 6,514,767 to Natan discloses SERS-active composite nanoparticles (SACNs) comprising a metal nanoparticle that has attached or associated with its surface one or more Raman-active molecules and is encapsulated by a shell comprising a polymer, glass, or any other dielectric material. U. S. Patent No. 5,714,389 describes lateral flow immunoassay methods and test devices using a colored particle that may be a metal colloid, preferably gold. Similarly, U. S. Patent No. 7,109,042 describes lateral flow immunoassay devices that use direct labels, such as gold sols and dye sols, which allow for the production of an instant analytical result without the need to add further reagents in order to develop a detectable signal. WO2007/090058 discloses lateral flow immunoassays using encapsulating metal particles, in particular encapsulated SERS nanoparticles, as the detection modality. An improved sensitivity of the lateral flow immunoassay prepared for visual reading was observed.

SERS is one of the most sensitive methods for performing chemical analyses, permitting detection of a single molecule. *See* Nie, S. and S. R. Emory, "Probing Single Molecules and Single Nanoparticles by Surface Enhanced Raman Scattering", Science, 275,1102 (1997). A Raman spectrum, similar to an infrared spectrum, includes a wavelength distribution of bands corresponding to molecular vibrations specific to the sample being analyzed (the analyte). In the practice of Raman spectroscopy, the beam from a light source, generally a laser, is focused upon the sample to thereby generate inelastically scattered radiation, which is optically collected and directed into a wavelength-dispersive or Fourier transform spectrometer in which a detector converts the energy of impinging photons to electrical signal intensity.

The very low conversion of incident radiation to inelastic scattered radiation limited Raman spectroscopy to applications that were difficult to perform by infrared spectroscopy, such as the analysis of aqueous solutions. It was discovered in 1974, however, that when a molecule in close proximity to a roughened silver electrode is subjected to a Raman excitation source, the intensity of the signal generated is increased by as much as six orders of magnitude. (Fleischmann, M., Hendra, P. J., and McQuillan, A. J., "Raman Spectra of Pyridine Adsorbed at a Silver Electrode," Chem. Phys. Lett, 26, 123, (1974), and Weaver, M. J., Farquharson, S., Tadayyoni, M. A., "Surface-enhancement factors for Raman scattering at silver electrodes. Role of adsorbate-surface interactions and electrode structure," J. Chem. Phys., 82, 4867 4874 (1985)). Briefly, incident laser photons couple to free conducting electrons within the metal which, confined by the particle surface, collectively cause the electron cloud to resonate. The resulting surface plasmon field provides an efficient pathway for the transfer of energy to the molecular vibrational modes of a molecule within the field, and thus generates Raman photons.

SERS nanoparticles have been used as a detection molecule in lateral flow immunoassays. For example, Oxonica (Kidlington, UK) has developed Nanoplex™ nanoparticles for use in such assays. The nanoparticles consist of a gold nanoparticle core, onto which are adsorbed Raman reporter molecules capable of generating a surface enhanced Raman spectroscopy signal. The Raman-labeled gold nanoparticle is coated with a silica shell of approximately 10-50 nm thickness. The silica shell protects the reporter from desorption from the surface, prevents plasmon-plasmon interactions between adjacent gold particles, and also prevents the generation of SERS signals from components in the solution. SERS nanoparticles having a polymer coating in place of the silica coating are described in U. S. Patent Application Publication No. 2007/0165219. WO00/31538 discloses lateral flow assays using colloidal gold as label and reflectance measurements for detection.

While taking advantage of the high sensitivity of SERS, detection of the signal produced from a SERS nanoparticle requires the use of an instrument capable of detecting a Raman signal. It may be advantageous in some circumstances to have a nanoparticle for use in a lateral flow immunoassay possessing increased sensitivity perhaps comparable to that of a SERS nanoparticle, but requiring only relatively simple and inexpensive reflectance reader technology for detection.

### SUMMARY

The invention provides rapid and accurate methods for determining qualitatively or quantitatively the presence or absence of analytes in biological samples and devices and reagents to perform those methods.

The inventors have discovered that coated nanoparticles used as a detector molecule in a lateral flow or vertical flow-through immunoassay provide significant assay sensitivity advantages over detector molecules such as colloidal gold when the assay is read with a reflectance reader. The invention provides
(1) a method for determining the presence or absence of an analyte in a liquid sample, comprising:
   (a) providing a test device for determining the presence or absence of an analyte in a liquid sample, comprising:
      (i) a sample receiving member;
      (ii) a carrier in fluid communication with the sample receiving member;
      (iii) a labeled reagent which is mobile in the carrier in the presence of the liquid sample, the labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, and wherein the coated nanoparticle does not include a Raman-active molecule; and
      (iv) a binding reagent effective to capture the analyte, when present, immobilized in a defined detection zone of the carrier;
   (b) contacting the liquid sample with the sample receiving member of the test device;
   (c) allowing the liquid sample applied to the sample receiving member to mobilize said labeled reagent such that the liquid sample and labeled reagent move along the length of the carrier to pass into the detection zone;
   (d) detecting the presence of the labeled reagent in the detection zone by measuring reflectance, wherein detection of the labeled reagent in the detection zone is indicative of the presence of analyte in the liquid sample, and failure to detect the presence of the labeled reagent in the detection zone is indicative of the absence of the analyte in the liquid sample;
(2) a method for determining the presence or absence of an analyte in a liquid sample, comprising:
   a) providing a test device comprising: (i) a sample receiving member; (ii) a carrier in fluid communication with the sample receiving member; and (iii) a binding reagent effective to capture the analyte, when present, immobilized in a defined detection zone of the carrier;
   b) mixing the liquid sample with a labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, wherein the coated nanoparticle does not include a Raman-active molecule;
   c) contacting the mixture of b) with the sample receiving member of the test device;
   d) allowing the mixture of b) applied to the sample receiving member to move along the length of the carrier to pass into the detection zone;
   e) detecting the presence of the labeled reagent in the detection zone by measuring reflectance, wherein detection of the labeled reagent in the detection zone is indicative of the presence of analyte in the liquid sample, and failure to detect the presence of the labeled reagent in the detection zone is indicative of the absence of the analyte in the liquid sample;
(3) a method for determining the presence or absence of an analyte in a liquid sample using a kit for performing a flow-through analytical test for detecting the presence or absence of an analyte in a liquid sample by reflectometry, said kit comprising:
   (i) a test device comprising a porous membrane comprising an upper surface and a lower surface and a binding reagent effective to capture the analyte, when present in the liquid sample, attached to the upper or lower surface of the porous membrane; and
   (ii) a labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, wherein the coated nanoparticle does not include a Raman-active molecule,
   said method comprising:
   (a) contacting the liquid sample with the upper surface of the porous membrane; (b) allowing the liquid sample to flow through the porous membrane such that at least a portion of the analyte, when present in the liquid sample, binds to the binding reagent;
   (c) contacting the labeled reagent with the upper surface of the porous membrane;
   (d) allowing the labeled reagent to flow through the porous membrane such that at least a portion of the labeled reagent binds to the analyte; and
   (e) detecting the presence of the labeled reagent on the porous membrane by measuring reflectance, wherein detection of the labeled reagent on the porous membrane is indicative of the presence of the analyte in the liquid sample, and failure to detect the presence of the labeled reagent on the porous membrane is indicative of the absence of the analyte in the liquid sample;
(4) a method for determining the presence or absence of an analyte in a liquid sample using a kit for performing a flow-through analytical test for detecting the presence or absence of an analyte in a liquid sample by reflectometry, said kit comprising:
   (i) a test device comprising a porous membrane comprising an upper surface and a lower surface and a binding reagent effective to capture the analyte, when present in the liquid sample, attached to the upper or lower surface of the porous membrane; and
   (ii) a labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, wherein the coated nanoparticle does not include a Raman-active molecule,
   said method comprising:
   (a) mixing the liquid sample with the labeled reagent such that the analyte, when present in the liquid sample, binds to the labeled reagent;
   (b) contacting the mixture of (a) with the upper surface of the porous membrane;
   (c) allowing the mixture of (a) to flow through the porous membrane such that at least a portion of the analyte bound to the labeled reagent binds to the binding reagent; and
   (d) detecting the presence of the labeled reagent on the porous membrane by measuring reflectance, wherein detection of the labeled reagent on the porous membrane is indicative of the presence of analyte in the liquid sample, and failure to detect the presence of the labeled reagent on the porous membrane is indicative of the absence of the analyte in the liquid sample;
(5) a system comprising
   (I) a test device for determining the presence or absence of an analyte in a liquid sample, comprising:
      (i) a sample receiving member;
      (ii) a carrier in fluid communication with the sample receiving member;
      (iii) a labeled reagent which is mobile in the carrier in the presence of the liquid sample, the labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, and wherein the coated nanoparticle does not include a Raman-active molecule; and
      (iv) a binding reagent effective to capture the analyte, when present, immobilized in a defined detection zone of the carrier; wherein the liquid sample applied to the sample receiving member mobilizes the labeled reagent such that the sample and labeled reagent are transported along the length of the carrier to pass into the detection zone, and wherein detection of the labeled reagent in the detection zone is indicative of the presence of analyte in the liquid sample, and
   (II) a reflectometer adapted to detect the presence of the labeled reagent in the test device; and
(6) a system comprising
   (I) a a kit for performing a flow-through analytical test for detecting the presence or absence of an analyte in a liquid sample by reflectometry, comprising:
      (i) a test device comprising a porous membrane comprising an upper surface and a lower surface and a binding reagent effective to capture the analyte, when present in the liquid sample, attached to the upper or lower surface of the porous membrane; and
      (ii) a labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, wherein the coated nanoparticle does not include a Raman-active molecule, and
   (II) a reflectometer adapted to detect the presence of the labeled reagent in the test device.

Such a coated nanoparticle comprises metal cores and a shell that increases the reflectance of the nanoparticle, wherein the coated nanoparticle does not include a Raman-active molecule. The metal cores may for example exhibit plasmon resonance such as, for example, gold.

In some embodiments, the shell comprises silica, while in other embodiments the shell is another ceramic material, such as another oxide, and in further embodiments the shell is comprised of a polymer. The polymer may be, for example, polyethylene glycol, polymethylmethacrylate, or polystyrene. The shell may completely or incompletely surround the core.

The coated nanoparticles may be formed in any of a variety of shapes having different dimensions, including but not limited to, spheroids, rods, disks, pyramids, cubes, cylinders, etc. In certain embodiments, the coated nanoparticle has at least one dimension in the range of about 1 nm to about 1000 nm. In other embodiments the core of the coated nanoparticle is spherical. In some cases, the diameter of the core is about 10-100 nm, while in other embodiments the diameter of the core is about 20-60 nm. In some embodiments, the nanoparticles comprise multi-core aggregates, for example but not limited to, doublets.

The shell of the nanoparticle is modified so as to allow for the conjugation of molecules to the surface of the nanoparticle. In particular embodiments, the modification introduces thiol groups onto the surface of the coated nanoparticle. In other embodiments, a ligand, for example and without limitation, an antibody, is conjugated to the shell of the coated nanoparticle via the thiol groups.

The ligand may bind to any analyte of interest that may or may not be present in a sample. In certain embodiments, the ligand is an antibody that binds to proteins specific to influenza virus A or influenza virus B.

In certain embodiments, the labeled reagent used in the test device comprises a ligand that binds to the analyte and a coated nanoparticle consisting of metal cores and a shell that increases the reflectance of the nanoparticle having the ligand attached thereto, wherein said ligand is bound to the surface of the shell.

In certain embodiments, the carrier is, for example and without limitations, nitrocellulose, plastic, or glass. In other embodiments, the test device further comprises an absorbent pad and/or a control zone in fluid communication with the detection zone.

The test device of the invention may be used to qualitatively or quantitatively detect the presence or absence of any analyte of interest, such as and without limitation, a protein, nucleic acid, metabolite, small molecule, virus, or bacterium. In certain embodiments, the test device may be used to detect multiple analytes in a liquid sample with at least two different labeled reagents, wherein the ligands of the labeled reagents bind to different analytes, and at least two detection zones for detecting each of the at least two different labeled reagents.

In some embodiments a reflectance reader is used to detect the labeled reagent in the detection zone. In some embodiments the analyte is detected quantitatively and in other embodiments, the analyte is detected qualitatively. In come embodiments, the methods of the invention can be used to detect multiple analytes in a liquid sample with at least two different labeled reagents, wherein the ligands of the labeled reagents bind to different analytes, and at least two detection zones for detecting each of the at least two different labeled reagents.

In certain embodiments, the test device of the kits described above further comprises an absorbent pad, wherein the lower surface of the porous membrane and the absorbent pad are in physical contact and in fluid communication, and wherein the binding reagent is attached to the upper surface of the porous membrane. In other embodiments, the test device of the kits of the invention further comprises a housing for the porous membrane.

The kits may be used to qualitatively or quantitatively detect the presence or absence of any analyte of interest, such as and without limitation, a protein, nucleic acid, metabolite, small molecule, virus, or bacterium. In certain embodiments, the kits may be used to detect multiple analytes in a liquid sample with at least two different labeled reagents, wherein the ligands of the labeled reagents bind to different analytes, and at least two detection zones for detecting each of the at least two different labeled reagents.

In some embodiments a reflectance reader is used to detect the labeled reagent in the detection zone. In some embodiments the analyte is detected quantitatively and in other embodiments, the analyte is detected qualitatively. In some embodiments, the methods of the
invention can be used to detect multiple analytes in a liquid sample with at least two different labeled reagents, wherein the ligands of the labeled reagents bind to different analytes, and at least two detection zones for detecting each of the at least two different labeled reagents.

### BRIEF DESCRIPTION OF THE DRAWINGS

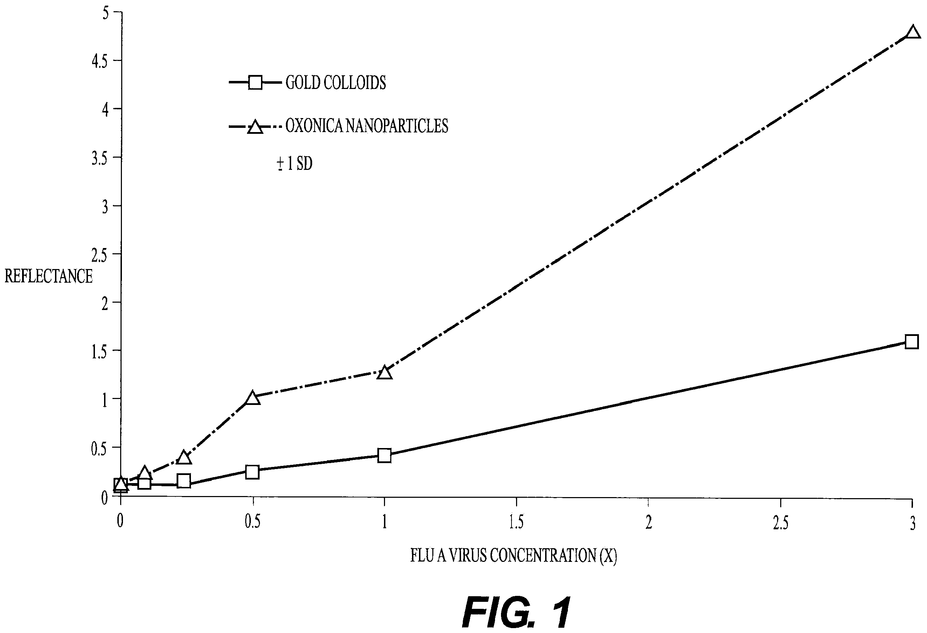

Figure 1 shows the results of a lateral flow test for influenza A virus comparing the sensitivity of colloidal gold and silica-coated gold nanoparticles when measured with a reflectometer. As seen in the Figure, a stronger response was observed for the silica-coated gold nanoparticles compared to the gold colloids.

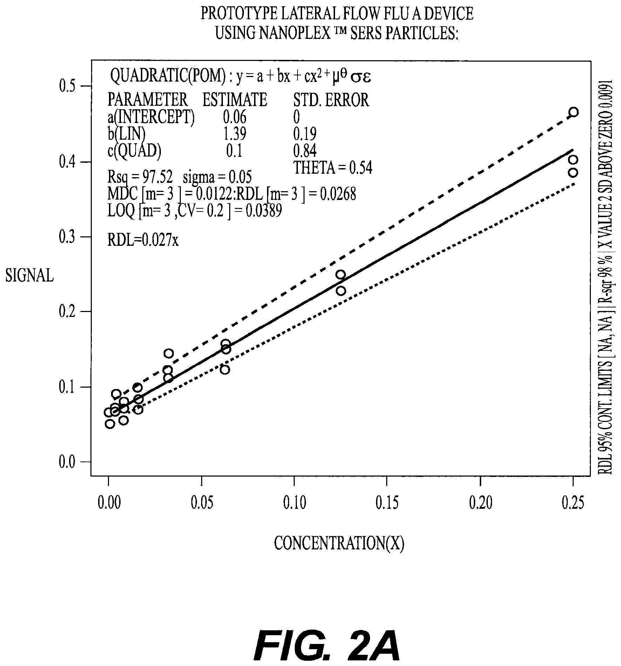

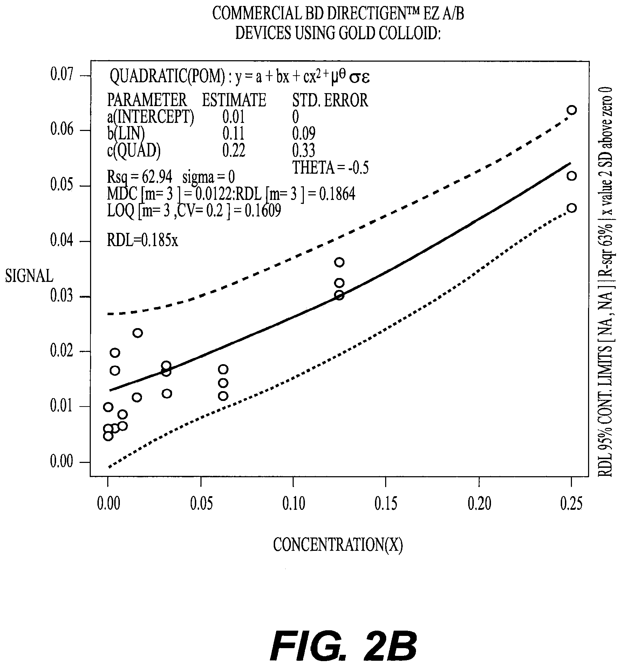

Figure 2 shows the results of a lateral flow test for influenza A virus comparing the sensitivity of a prototype device using silica-coated gold nanoparticles (Panel 2A) with the sensitivity of a commercial product, BD Directigen™ EZ A+B (Panel 2B), when both devices are read with a reflectometer. The device using the silica-coated gold nanoparticles is approximately 7-fold more sensitive than the BD Directigen™ EZ A+B when read with a reflectometer.

### DETAILED DESCRIPTION OF CERTAIN EMBODIMENTS

Unless otherwise defined, all technical and scientific terms used herein have the same meaning as commonly understood by one of ordinary skill in the art. Although methods and materials similar or equivalent to those described herein can be used in the practice of the claims, suitable methods and materials are described below. In the case of conflict, the present specification, including definitions, will control. In addition, the materials, methods, and examples are illustrative only and not intended to be limiting.

In this application, the use of the singular includes the plural unless specifically stated otherwise. In this application, the use of "or" means "and/or" unless stated otherwise. In the context of a multiple dependent claim, the use of "or" refers back to more than one preceding independent or dependent claim in the alternative only. Furthermore, the use of the term "including," as well as other forms, such as "includes" and "included," is not limiting. Also, terms such as "element" or "component" encompass both elements and components comprising one unit and elements and components that comprise more than one subunit unless specifically stated otherwise.

Other features and advantages will be apparent from the following detailed description and claims.

In order that the present application may be more readily understood, certain terms are first defined. Additional definitions are set forth throughout the detailed description.

The term "nanoparticle" as used herein, refers to particles comprising at least one core and a shell having one dimension in the range of about 1 to about 1000 nanometers ("nm"). The nanoparticles may be of any shape. In certain embodiments the nanoparticles are spherical. The nanoparticles described typically do not, but can, include a Raman-active molecule. In certain embodiments, the nanoparticles may comprise multiple cores and one shell.

As used herein the term "core" refers to the internal portion of the nanoparticles. In the invention the metal cores are, for example gold.

The nanoparticles also comprise a shell that enhances the reflectance of the nanoparticles. The shell may completely encapsulate the core, or incompletely encapsulate the core. The shell may be composed of any material or combination of materials, as long as it possesses the property of enhancing reflectance of the nanoparticle. For example, in some embodiments the shell may comprise any material transparent in the required spectral range. In certain embodiments, the shell comprises silica, that is, glass. In other embodiments, the shell comprises another ceramic material, for example but not limited to, transparent ceramics with a high refractive index, such as perovskite and ZrO₂. In other embodiments, the shell is composed of a polymer. In particular embodiments the polymer comprises polyethylene glycol, polymethylmethacrylate, or polystyrene. In some embodiments, the material forming the shell is treated or derivitized to permit attaching a ligand to the surface of the nanoparticle. The optimal choice of polymer may depend on the ligand being immobilized on the surface of the nanoparticle.

In referring to the shell of the present disclosure, the phrase "increases the reflectance of the nanoparticle" means that the presence of the shell results in a nanoparticle providing increased signal or sensitivity when measured by reflectance in, for example, a lateral flow immunoassay, as compared to a nanoparticle without the shell. Without being bound by theory, the presence of the shell surrounding the core may directly cause the particle to reflect more light. Alternatively, or in addition, ligands bound to the surface of the shell may be better oriented to participate in binding reactions when bound to the shell material as opposed to when passively adsorbed to the surface of the core.

As used herein, "ligand" means a molecule of any type that will bind to an analyte of interest. For example and without limitation, in certain embodiments the ligand is an antibody, an antigen, a receptor, a nucleic acid, or an enzyme.

The term "analyte" as used herein refers to any substance of interest that one may want to detect using the invention, including but not limited to drugs, including therapeutic drugs and drugs of abuse; hormones; vitamins; proteins, including antibodies of all classes; peptides; steroids; bacteria; fungi; viruses; parasites; components or products of bacteria, fungi, viruses, or parasites; allergens of all types; products or components of normal or malignant cells; etc. As particular examples, there may be mentioned human chorionic gonadotropin (hCG); insulin; luteinizing hormone; organisms causing or associated with various disease states, such as *Streptococcus pyogenes* (group A), Herpes Simplex I and II, cytomegalovirus, *Chlamydia*, rubella antibody, influenza A and B; etc. In certain embodiments of the invention, the presence or absence of an analyte in a sample is determined qualitatively. In other embodiments, a quantitive determination of the amount or concentration of analyte in the sample is determined.

The term "sample" as used herein refers to any biological sample that could contain an analyte for detection. In some embodiments, the biological sample is in liquid form, while in others it can be changed into a liquid form.

The term "sample receiving member" as used herein means the portion of the test device which is in direct contact with the liquid sample, that is, it receives the sample to be tested for the analyte of interest. The sample receiving member may be part of, or separate from, the carrier or porous membrane. The liquid sample can then migrate, through lateral or vertical flow, from the sample receiving member towards the detection zone. The sample receiving member is in liquid flow contact with the analyte detection zone. This could either be an overlap, top-to-bottom, or an end-to-end connection. In certain embodiments, the sample receiving member is made of porous material, for example and not limited to, paper.

As used herein, the term "carrier," such as used in a lateral flow assay, refers to any substrate capable of providing liquid flow. This would include, for example, substrates such as nitrocellulose, nitrocellulose blends with polyester or cellulose, untreated paper, porous paper, rayon, glass fiber, acrylonitrile copolymer, plastic, glass, or nylon. The substrate may be porous. Typically, the pores of the substrate are of sufficient size such that the nanoparticles flow through the entirety of the carrier. One skilled in the art will be aware of other materials that allow liquid flow. The carrier may comprise one or more substrates in fluid communication. For example, the reagent zone and detection zone may be present on the same substrate (*i.e*., pad) or may be present on separate substrates (*i.e*., pads) within the carrier.

As used herein, "porous membrane," such as used in a flow through assay, refers to a membrane or filter of any material that wets readily with an aqueous solution and has pores sufficient to allow the coated nanoparticles to pass through. Suitable materials include, for example, nitrocellulose, nitrocellulose blends with polyester or cellulose, untreated paper, porous paper, rayon, glass fiber, acrylonitrile copolymer, plastic, glass, or nylon.

As used herein, "absorbent material" refers to a porous material having an absorbing capacity sufficient to absorb substantially all the liquids of the assay reagents and any wash solutions and, optionally, to initiate capillary action and draw the assay liquids through the test device. Suitable materials include, for example, nitrocellulose, nitrocellulose blends with polyester or cellulose, untreated paper, porous paper, rayon, glass fiber, acrylonitrile copolymer, plastic, glass, or nylon.

As used herein the term "lateral flow" refers to liquid flow along the plane of a carrier. In general, lateral flow devices may comprise a strip (or several strips in fluid communication) of material capable of transporting a solution by capillary action, *i.e*., a wicking or chromatographic action, wherein different areas or zones in the strip(s) contain assay reagents either diffusively or non-diffusively bound that produce a detectable signal as the solution is transported to or through such zones. Typically, such assays comprise an application zone adapted to receive a liquid sample, a reagent zone spaced laterally from and in fluid communication with the application zone, and an detection zone spaced laterally from and in fluid communication with the reagent zone. The reagent zone may comprise a compound that is mobile in the liquid and capable of interacting with an analyte in the sample and/or with a molecule bound in the detection zone. The detection zone may comprise a binding molecule that is immobilized on the strip and is capable of interacting with the analyte and/or the reagent compound to produce a detectable signal. Such assays may be used to detect an analyte in a sample through direct (sandwich assay) or competitive binding. Examples of lateral flow devices are provided in U.S. Patent Nos. 6,194,220 to Malick et al.; 5,998,221 to Malick et al.; 5,798,273 to Shuler et al.; and RE38,430 to Rosenstein.

In a sandwich lateral flow assay, a liquid sample that may or may not contain an analyte of interest is applied to the application zone and allowed to pass into the reagent zone by capillary action. The analyte, if present, interacts with a labeled reagent in the reagent zone and the analyte-reagent complex moves by capillary action to the detection zone. The analyte-reagent complex becomes trapped in the detection zone by interacting with a binding molecule specific for the analyte and/or reagent. Unbound sample may move through the detection zone by capillary action to an absorbent pad laterally juxtaposed and in fluid communication with the detection zone. The labeled reagent may then be detected in the detection zone by appropriate means.

In a competitive lateral flow assay, a liquid sample that may or may not contain an analyte of interest is applied to the application zone and allowed to pass into the reagent zone by capillary action. The reagent zone comprises a labeled reagent, which may be the analyte itself, a homologue or derivative thereof, or a moiety that is capable of mimicking the analyte of interest when binding to an immobilized binder in the detection zone. The labeled reagent is mobile in the liquid phase and moves with the liquid sample to the detection zone by capillary action. The analyte contained in the liquid sample competes with the labeled reagent in binding to the immobilized binder in the detection zone. Unbound sample may move through the detection zone by capillary action to an absorbent pad laterally juxtaposed and in fluid communication with the detection zone. The labeled reagent may then be detected in the detection zone by appropriate means. The presence or absence of the analyte of interest may be determined through inspection of the detection zone, wherein the greater the amount of analyte present in the liquid sample, the lesser the amount of labeled receptor bound in the detection zone.

As used herein, the terms "vertical flow" and "flow through" refer to liquid flow transverse to the plane of a carrier. In general, flow through devices may comprise a membrane or layers of membranes stacked on top of each other that allow the passage of liquid through the device. The layers may contain assay reagents either diffusively or non-diffusively bound that produce a detectable signal as the solution is transported through the device. Typically, the device comprises first layer having an upper and lower surface, wherein said upper surface is adapted to receive a liquid sample, and an absorbent layer vertically juxtaposed and in fluid communication with the lower surface of the first layer that is adapted to draw the liquid sample through the first layer. The first layer may comprise a binding agent attached to the upper surface of the first layer that is capable of interacting with an analyte in the sample and trapping the analyte on the upper surface of the first layer. Examples of flow through devices are provided in U.S. Patent Nos. 4,920,046 to McFarland et al. and 7,052,831 to Fletcher et al.

In practice, a liquid sample that may or may not contain an analyte of interest is applied to the upper surface of a first layer comprising a binding agent specific for an analyte of interest. The liquid sample then flows through the first layer and into the absorbent layer. If analyte is present in the sample, it interacts with the binding agent and is trapped on the upper surface of the first layer. The first layer may then be treated with wash solutions in accordance with conventional immunoassay procedures. The first layer may then be treated with a labeled reagent that binds to the analyte trapped by the binding agent. The labeled reagent then flows through the first layer and into the absorbent layer. The first layer may be treated with wash solutions in accordance with conventional immunoassay procedures. The labeled reagent may then be detected by appropriate means. Alternatively, the liquid sample may be mixed with the labeled reagent before being applied to the upper surface of the first layer. Other suitable variations are known to those skilled in the art.

Lateral and flow through assays may be used to detect multiple analytes in a sample. For example, in a lateral flow assay, the reagent zone may comprise multiple labeled reagents, each capable of binding to (or mimicking) a different analyte in a liquid sample, or a single labeled reagent capable of binding to (or mimicking) multiple analytes. Alternatively, or in addition, the detection zone in a lateral flow assay may comprise multiple binding molecules, each capable of binding to a different analyte in a liquid sample, or a single binding molecule capable of binding to multiple analytes. In a flow through assay, the porous membrane may comprise multiple binding agents, each capable of binding to a different analyte in a liquid sample, or a single binding agent capable of binding to multiple analytes. Alternatively, or in addition, a mixture of labeled reagents may be used in a flow through assay, each configured to bind to a different analyte in a liquid sample, or a single labeled reagent configured bind multiple analytes. If multiple labeled reagents are used in a lateral or flow through assay, the reagents may be differentially labeled to distinguish different types of analytes in a liquid sample.

As used herein, the term "mobile" means diffusively or non-diffusively attached, or impregnated. The reagents which are mobile are capable of dispersing with the liquid sample and are carried by the liquid sample in the lateral or vertical flow.

As used herein, the term "labeled reagent" means any particle, protein, or molecule which recognizes or binds to the analyte of interest and has attached to it a substance capable of producing a signal that is detectable by visual or instrumental means, that is, a coated nanoparticle as defined herein. The particle or molecule recognizing the analyte can be either natural or non-natural. In some embodiments the molecule is a monoclonal or polyclonal antibody.

As used herein, the term "binding reagent" means any particle or molecule which recognizes or binds the analyte in question. The binding reagent is capable of forming a binding complex with the analyte-labeled reagent complex. The binding reagent is immobilized to the carrier in the detection zone or to the surface of the porous membrane. The binding reagent is not affected by the lateral or vertical flow of the liquid sample due to the immobilization to the carrier or porous membrane. The particle or molecule can be natural, or non-natural, that is, synthetic. Once the binding reagent binds the analyte-labeled reagent complex it prevents the analyte-labeled reagent complex from continuing with the flow of the liquid sample.

As used herein, "detection zone" means the portion of the carrier or porous membrane containing the immobilized binding reagent.

The term "control zone" refers to a portion of the test device comprising a binding molecule configured to capture the labeled reagent. In a lateral flow assay, the control zone may be in liquid flow contact with the detection zone of the carrier, such that the labeled reagent is captured in the control zone as the liquid sample is transported out of the detection zone by capillary action. In a flow through assay, the control zone may be a separate portion of the porous membrane, such that the labeled reagent is applied both to the sample application portion of the porous membrane and the control zone. Detection of the labeled reagent in the control zone confirms that the assay is functioning for its intended purpose.

The term "housing" refers to any suitable enclosure for the test devices of the invention. Exemplary housings will be known to those skilled in the art. The housing may have, for example, a base portion and a lid portion. The lid may include a top wall and a substantially vertical side wall. A rim may project upwardly from the top wall. The rim may define a recess having therein an insert with at least two openings in alignment with at least two other openings in the lid to form at least two wells in the housing. The housing may be constructed to ensure that there is no communication between the two or more wells. An example of such a housing is provided in U.S. Patent No. 7,052,831 to Fletcher et al. Other suitable housings include those used in the BD Directigen™ EZ RSV lateral flow assay device.

As used herein, "reflectance reader" or "reflectometer" refers to an instrument capable of detecting the change in reflectance caused the by presence of the coated nanoparticle in the detection zone of the test device. Reflectance readers or reflectometers are known in the art. Representative instruments suitable for use in the invention include, but are not limited to the Immunochromato Reader C10066 from Hamamatsu or the ESE-Quant from ESE GmbH. The detection zone is most commonly scanned by the detection area of the device or directly imaged on the detector of the reflectometer leading to a trace of reflectivity versus spatial coordinate. A suitable algorithm is then used to determine, for example, the maximum change of reflectivity in the detection zone.

Coated nanoparticles for use in SERS-based methods are known in the art. For example, U.S. Patent No. 6,514,767 describes SERS-active composite nanoparticles (SACNs) comprising a metal nanoparticle that has attached or associated with its surface one or more Raman-active molecules and is encapsulated by a shell comprising a polymer, glass, or any other dielectric material. Such particles may be produced by growing or otherwise placing a shell of a suitable encapsulant over a Raman-active metal nanoparticle core. Metal nanoparticles of the desired size can be grown as metal colloids by a number of techniques well known in the art, such as chemical or photochemical reduction of metal ions in solution using reducing agents. For example, colloidal gold particles, which are suspensions of sub-micrometer-sized particles of gold in fluid, may be produced in a liquid by reduction of chloroauric acid. After dissolving the acid, the solution is rapidly stirred while a reducing agent is added. This causes gold ions to be reduced to neutral gold atoms, which precipitate from the supersaturated solution and form particles. To prevent the particles from aggregating, a stabilizing agent that sticks to the nanoparticle surface may be added. Nanoparticles can also be made by electrical discharge in solution. The particles can be functionalized with various organic ligands to create organic-inorganic hybrids with advanced functionality.

Suitable encapsulants include glass, polymers, metals, metal oxides, and metal sulfides. If the encapsulant is glass, the metal nanoparticle cores are preferably treated first with a glass primer. Glass is then grown over the metal nanoparticle by standard techniques. The thickness of the encapsulant can be easily varied depending on the physical properties required of the particle. The shells may be derivatized by standard techniques, allowing the particles to be conjugated to molecules (including biomolecules such as proteins and nucleic acids) or to solid supports.

Commercially available Oxonica Nanoplex™ nanoparticles consist of a gold nanoparticle core, onto which are adsorbed Raman reporter molecules capable of generating a SERS signal. The Raman-labeled gold nanoparticle is then coated with a silica shell of approximately 10-50 nm thickness. The silica shell protects the reporter molecules from desorption from the surface of the core, prevents plasmon-plasmon interactions between adjacent gold particles, and also prevents the generation of SERS signals from components in the solution.

For a lateral flow assay read with a reflectance reader, one might expect the Oxonica Nanoplex™ nanoparticles to give an assay sensitivity comparable to that of gold colloids of the same diameter as the Oxonica Nanoplex™ nanoparticle's core and used at the same optical density. Instead, the inventors observed unexpected and significant advantages in assay sensitivity when gold colloids are replaced by Oxonica Nanoplex™ nanoparticles in lateral flow assays read with a reflectance reader instead of a Raman signal detector. Importantly, the sensitivity improvements obtained with the Oxonica Nanoplex™ nanoparticles were generally not obtainable merely by adjusting the diameter of the uncoated gold colloids. Also, importantly, when the Oxonica Nanoplex™ nanoparticles are used as a labeled reagent, the sensitivity of the assay may be equivalent, whether the signal is read with a reflectometer or a SERS reader.

The Raman-active molecules in the Oxonica Nanoplex™ nanoparticles are not believed to contribute to the assay performance when a reflectometer is used to read the signal rather than a Raman reader. This expectation is borne out by experiments in which silica-coated nanoparticle (without Raman-active molecules) was found to perform identically and have been used in a reflectometer-based lateral flow assays to give significant sensitivity advantages over bare gold colloids.
Those of the following examples not falling within the scope of the claims are for illustrative purpose.

### EXAMPLES

### Example 1:

This experiment compared the performance of silica-coated gold nanoparticles to that of gold colloids in a lateral flow immunoassay for influenza A virus. In this experiment, nasopharyngeal aspirates that tested negative for influenza A virus were spiked with known amounts of a live H1N1 influenza A virus.

The gold colloids, obtained from British Biocell International ("BBInternational"; "BBI"), were 40 nm in diameter. The silica-coated gold nanoparticles used in the experiment were Oxonica Nanoplex™ nanoparticles. These nanoparticles consist of a 60 nm gold core coated with the Raman reporter 4,4'-Dipyridyl and an outer silica shell. The silica shell thickness was approximately 30 nm. Influenza A detection antibodies were attached to the gold colloids by passive adsorption. For the Nanoplex™ nanoparticles, the influenza A detection antibodies were covalently bound to thiol groups on the silica surface through maleimide chemistry. For both assays, influenza A capture antibodies were striped onto Whatman AE99 nitrocellulose membranes. The nitrocellulose membranes were then assayed in a liquid ("dipstick") format in which the particles plus detection antibodies were mixed with the nasopharyngeal samples containing varying levels of virus. The same optical density was used for both the gold colloids and the coated nanoparticles. The particle/sample mixtures were then placed in the wells of a 96-well plate, and one end of a nitrocellulose membrane with capture antibodies was placed in each well. The sample was allowed to wick up the nitrocellulose membrane, and the wells were then filled with a wash buffer that then also wicked up the strips of nitrocellulose membrane.

The resulting signal from the capture line was read with a reflectometer custom-built for Becton, Dickinson and Company ("BD") by UMM Electronics (Indianapolis, IN). Figure 1 shows a plot of reflectometer signal as a function of virus concentration for lateral flow tests with gold colloids and with coated nanoparticles. A significantly stronger response was seen for the Oxonica nanoparticles versus the gold colloids.

### Example 2:

In this example, the sensitivity of silica-coated gold nanoparticles was compared with that of a commercial product: the BD Directigen™ EZ Flu A+B test. As in Example 1, the coated gold nanoparticles were assayed in a dipstick format using Whatman AE99 nitrocellulose. The Directigen™ EZ Flu A+B was tested in its commercial embodiment ("cartridge" format). Capture and detection antibodies were the same for both the BD Directigen™ EZ Flu A+B commercial product and the Oxonica Nanoplex™ nanoparticles-based device. Samples were prepared as described in Example 1, except live B/Lee/40 influenza B virus was spiked into negative nasopharyngeal aspirates rather than influenza A virus. As before, the silica-coated gold nanoparticles used in the experiment were Oxonica Nanoplex™ nanoparticles. These nanoparticles consist of a 60 nm gold core coated with the Raman reporter 4,4'-Dipyridyl and an outer silica shell. The silica shell thickness was approximately 30 nm. The BD commercial product uses 40 nm gold colloid that does not include a silica shell. The BD commercial product was used according to the package insert, only with the test line signal read with a reflectometer as well as visually.

The data are shown in Table 1, which compares the limit of detection (Reliable Detection Limit) for the two particles. The Reliable Detection Limit (RDL) is defined as the concentration where the lower 95% confidence limit equals the upper 95% confidence limit of the blank. The RDLs, expressed in arbitrary units (X) for the BD colloidal gold product and for silica-coated nanoparticles, are reported for three separate experiments. The same custom-built reflectometer from UMM Electronics was used to read the signal from both the silica-coated nanoparticle dipstick devices and the Directigen™ EZ Flu A+B devices. The Sensitivity Improvement Factor is defined as the RDL of the uncoated gold-based product divided by the RDL of the silica-coated nanoparticle product.

**Table 1**

| | **BD Directigen™ EZ with uncoated gold (read with a reflectometer)** | **Lateral flow test with Oxonica nanoparticles (read with a reflectometer)** | **Sensitivity Improvement Factor** |
|---|---|---|---|
| **Experiment 1** | 0.23 | 0.015 | 15 |
| **Experiment 2** | 0.12 | 0.0075 | 16 |
| **Experiment 3** | 0.26 | 0.028 | 9 |

As seen from the table, the Oxonica Nanoplex™ nanoparticles gave up to 16-fold improved sensitivity compared to bare gold particles.

In separate experiments similar to those described above, the diameter of the gold colloid particles was increased from 40 nm to 60 nm and larger sizes. In these experiments, only limited sensitivity improvements (up to 2-fold) were observed, indicating that the difference in size between the gold core of the Oxonica Nanoplex™ nanoparticles and the gold colloid is likely not the source of the sensitivity improvements.

### Example 3:

This experiment compared the sensitivity and specificity of gold colloids versus Oxonica Nanoplex™ nanoparticles in an influenza B (B/Lee/40) lateral flow immunoassay test. Sixty clinical samples were prepared. The samples were all nasopharyngeal samples that tested negative for influenza B. Twenty of the samples served as negative controls. The remaining 40 samples were spiked with live influenza B virus at two levels to create a set of 40 positive samples. Twenty of these forty samples were spiked with live influenza B virus at a concentration of 0.5X (arbitrary units). The remaining twenty samples received 10-fold less flu B virus, for a concentration of 0.05X. All sixty samples (40 positives and 20 negative controls) were then tested using the BD Directigen™ EZ Flu A+B commercial product in cartridge format and the dipstick device made using Oxonica Nanoplex™ nanoparticles. The dipstick device was prepared using Whatman AE99 nitrocellulose. The Oxonica Nanoplex™ nanoparticles consisted of a 60 nm gold core coated with the Raman reporter 4,4'-Dipyridyl and an outer silica shell that was approximately 30 nm thick. Capture and detection antibodies were the same for both the BD Directigen™ EZ Flu A+B commercial product and the Oxonica Nanoplex™ nanoparticles-based device.

The results are summarized in Table 2. In the table, sensitivity was calculated as the percentage of the 40 positive samples correctly identified as positive by each test. Specificity was calculated as the percentage of the 20 negative samples correctly identified as negative by each test. When instrument readings were used, a test was called positive if the measured instrument reading was greater than an established threshold value for each type of device. The threshold was established to ensure at least a 95% specificity for each device. In this example, the Directigen™ product was read visually and with the custom-built reflectometer from UMM Electronics. The Oxonica Nanoplex™ nanoparticles were read with the same reflectometer, and also with a research Raman reader built by BD (last row of the table). The research Raman reader used a 785 nm laser for excitation and an Acton Research Spectrometer (SpectraPro 25000i) and a CCD detector (Pixis 400) for detecting the Raman signal.

**Table 2**

| **Test** | **Sensitivity** | **Specificity** |
|---|---|---|
| Directigen™ EZ, visual read | 45% | 100% |
| Directigen™ EZ, reflectometer read | 58% | 95% |
| Oxonica nanotags, reflectometer read | 100% | 100% |
| Oxonica nanotags, Raman read | 100% | 100% |

As can be seen, a clear sensitivity advantage is seen with the Oxonica Nanoplex™ nanoparticles compared to the Directigen™ EZ particles, without loss of specificity.

### Example 4:

This experiment compared the performance of silica-coated gold nanoparticles, with and without a surface enhanced Raman scattering (SERS) molecule, in a reflectance-based lateral flow immunoassay.

Oxonica Nanoplex™ gold nanoparticles containing a SERS tag were obtained from Oxonica. The nanoparticles consist of a 60 nm gold core tagged with 4-4'-Dipyridyl and coated with a 35 nm thick silica shell. The diameter of the nanoparticles was 130 nm. Sulfo-SMCC chemistry (Pierce #22622) was used to covalently attach anti-influenza A antibodies to surface thiol groups on the nanoparticles..

Sixty-nanometer diameter gold nanoparticles lacking a SERS tag were purchased from BBI. The gold nanoparticles were then coated with silica by the following process. 10 mL of 60 nm gold colloid (BBI, ∼2.6x10¹⁰ particles/mL) was first treated with 75 µL of a 100 µM solution of 3-mercaptopropyl triethoxysilane in ethanol. After 3 hours, 75 µL of a 2.7% aqueous solution of sodium silicate was added, and the reaction was allowed to continue for 24 hours. In the next step, 40 mL of ethanol was added to the suspension followed by 1 mL of ammonia and 300 µL (5% solution in ethanol) of tetraethyl orthosilicate. The reaction was kept under agitation for 2 days and finally purified by repeated centrifugation. The thickness of the silica coating produced in this process was 30 nm, for a total particle diameter of the silica-coated gold of 120 nm. Thiol groups were then added to the surface of the silica-coated gold particles by reacting an aqueous suspension of glass-coated gold nanoparticles (10 mL, ∼2.6x10¹⁰ particles/mL) with a 1% ethanolic solution of mercaptopropyl trimethoxysilane (MPTMS) for 24 hours at room temperature. The amount of MPTMS solution varied from 25 µL to 100 µL, to create varying levels of surface-active thiol groups at approximate loading levels of 0.5, 1.0, 1.5, and 2.0 relative to each other (for example, 2.0 is 4X greater than 0.5) through a non-optimized process. After the reaction, the particles were purified by repeated centrifugation in water. Sulfo-SMCC chemistry (Pierce #22622) was used to covalently attach anti-influenza A antibodies to surface thiol groups on the nanoparticles.

The lateral flow immunoassay was performed in a dipstick format as follows. Influenza A capture antibodies were applied to Whatman AE99 nitrocellulose strips. The nitrocellulose strips were dipped into sample solutions containing nanoparticles adjusted to an optical density of 10 and various concentrations of influenza A H1N1 virus. The sample solutions were allowed to completely wick up the nitrocellulose strips. A wash buffer was then added and also wicked up the strips of nitrocellulose membrane. The strips were then dried, and the reflectance was read using a Hamamatsu reflectometer, model C10066. The results are summarized in Table 3.

**Table 3**

| Nanoparticle Type | LOD - Reflectance Reader (units of X, measured as RDL) |
|---|---|
| Oxonica Nanoplex™ SERS tag with 4,4'-dipyridyl Raman reporter molecule - replicate 1 | 0.0471 |
| Oxonica Nanoplex™ SERS tag with 4,4'-dipyridyl Raman reporter molecule - replicate 2 | 0.0564 |
| Silica-coated gold with no Raman reporter molecule - thiol loading "0.5" | 0.0901 |
| Silica-coated gold with no Raman reporter molecule - thiol loading "1.0" | 0.135 |
| Silica-coated gold with no Raman reporter molecule - thiol loading "1.5" | 0.1063 |
| Silica-coated gold with no Raman reporter molecule - thiol loading "2.0" | 0.062 |

The data in Table 3 indicate that, if optimized, silica-coated gold nanoparticles without a SERS reporter perform as well as nanoparticles that include a SERS reporter in a lateral flow immunoassay when a reflectometer reader is used. Furthermore, the performance of the silica-coated gold particles may depend on the extent of thiolation.

### Example 5:

This experiment was devised to test the performance of a prototype lateral flow device for detecting live H1N1 influenza A virus in clinical samples using Oxonica Nanoplex™ silica-coated gold nanoparticles as the reporter. The Oxonica particles had a gold core diameter of approximately 60 nm, a Raman reporter of 4,4'-Dipyridyl, and an outer silica shell approximately 35 nm thick. The cartridge-based prototype device used the same capture and detection antibodies as the BD Directigen™ EZ Flu A+B commercial product and was used in the same manner as the commercial product.

The prototype test device comprised a backer strip supporting a length of Millipore HF135 nitrocellulose lateral flow membrane. Capture antibodies specific to influenza A nucleoprotein (Flu A NP) were striped across this membrane to form a test line. Anti-species immunoglobulin antibody was striped adjacent to the test line to form a control line. Oxonica Nanoplex™ SERS nanoparticles were sprayed onto a conjugate pad (Arista MAPDS-0399) that had been treated with a 10% SEABLOCK solution (Pierce 37527). The nanoparticles were conjugated with a detection antibody to Flu A NP. The conjugate pad was adhered to the backer strip at one end of the lateral flow membrane. At the opposite end of the lateral flow membrane, an absorbent wicking pad (Whatman #470) was attached. The resulting lateral flow assay strip was mounted within a two-part polystyrene cartridge. This cartridge completely enclosed the assay strip except for a central window revealing the test and control line region of the LF membrane, and a sample application well centered on the conjugate pad. This cartridge housing is used in the BD Directigen™ EZ RSV lateral flow assay device.

Similarly to Example 1, pediatric nasopharyngeal aspirate samples testing negative for influenza A virus were spiked with known amounts of live influenza A virus. Final virus concentrations within the spiked samples ranged from 0.25X (arbitrary units) down to 0.0039X by two-fold serial dilution. A sample spiked with virus-free dilution medium ("0X") was included as a control. After the addition of live virus, the series of samples was processed using the BD Directigen™ EZ Flu A+B sample preparation protocol. Briefly, a quantity of influenza A-spiked sample was mixed with extraction reagent in a flexible sample tube. The extracted sample was then expressed from the tube through a glass-fiber filtration tip and collected. Each sample was tested in triplicate by applying 100 µL to the sample well of each of three prototype test devices.

Each device made with the Oxonica Nanoplex™ SERS nanoparticles was read using a Hamamatsu reflectometer (model C10066) at both 15 minutes and 30 minutes following sample application. After the 30 minute read, each lateral flow strip was removed from its cartridge, stripped of conjugate and wicking pads, and dried at ambient temperature and humidity for at least 1 hour. Each strip was read again by reflectometer after drying. A dose-response curve was plotted using data from each read-time and then used to calculate sensitivity of each read-time in terms of minimum detectable concentration (the lowest concentration for which the mean signal equals the upper confidence interval of the blank; MDC) and reliable detection limit (RDL). These results are shown in Table 4. Table 4 also shows the sensitivity improvement obtained using the Oxonica particles compared to the sensitivity of Directigen™ EZ. Directigen™ EZ uses 40 nm-diameter gold colloids without a silica coating.

**Table 4. Sensitivity of a Cartridge-Based Flu A Lateral Flow Test System Employing Oxonica SERS-Active Nanoparticles and Detection by Reflectometry**

| **Device Status and Time of Read** | **Flu A Limit of Detection^{†} by Reflectometer Read (Oxonica particles)** | | **RDL Improvement vs. Directigen™ EZ Flu A Device read with a reflectometer** |
|---|---|---|---|
| | **MDC (X)** | **RDL** (**X**) | |
| Wet at 15 minutes | 0.0341 | 0.0666 | 3.0-fold |
| Wet at 30 minutes | 0.0131 | 0.0264 | 7.6-fold |
| Dried post-30 minutes | 0.0130 | 0.0242 | 8.3-fold |

| | | | |
|---|---|---|---|
| ^{†} Expressed as minimum detectable concentration (MDC) and reliable detection limit (RDL) | | | |

The limit of detection (RDL) for the current Directigen™ EZ Flu A device is approximately 0.5X Flu A virus concentration by visual read, and approximately 0.2X by reflectometer read. As shown in Table 4, the cartridge-based test device using Oxonica Nanoplex™ reporter nanoparticles provides a marked increase in sensitivity relative to the current Directigen™ EZ Flu A device.

### Example 6:

In this example, the analytical sensitivity of a prototype lateral flow Flu A test using Oxonica Nanoplex™ silica-coated gold nanoparticles was compared to the analytical sensitivity of the BD Directigen™ EZ Flu A+B commercial product read with a reflectometer. The Oxonica particles had a gold core diameter of approximately 60 nm, Raman reporter molecule 4,4'-Dipyridyl attached to the gold surface, and a silica shell thickness of approximately 35 nm. In this example, the amount of SERS nanoparticles per lateral flow device was estimated to be slightly less than the amount of gold colloid particles in the commercial product. Capture and detection antibodies were the same for both the BD Directigen™ EZ Flu A+B commercial product and the Oxonica Nanoplex™ nanoparticles-based device.

As in example 5, pediatric nasopharyngeal aspirate samples testing negative for influenza A virus were spiked with known amounts of live influenza A virus. Optimized prototype devices were assembled as described in example 5, and sample extraction was performed according to the BD Directigen™ EZ Flu A package insert. All devices (SERS-based prototypes and BD Directigen™ EZ Flu A+B) were read 15 minutes after sample application in a Hamamatsu reflectometer (model C10066). Figure 2 shows the reflectance signal as a function of live virus concentration for both assay formats, and Table 5 shows the reflectometer signal obtained at the highest test virus concentration (0.25X) along with the reliable detection limit (RDL) for the assays. In this experiment, devices using the SERS nanoparticles gave approximately 7-fold improved sensitivity and 8-fold improved brightness compared to BD Directigen™ EZ Flu A+B read with a reflectometer.

**TABLE 5**

| | Prototype (using Nanoplex™ SERS particles) | BD Directigen™ EZ A+B (using gold colloid) |
|---|---|---|
| RDL (arbitrary units) | 0.027x | 0.185x |
| Reflectance signal intensity at 0.25x (arb. Units) | 0.419 | 0.054 |

## Claims

1. A method for determining the presence or absence of an analyte in a liquid sample, comprising:
(a) providing a test device for determining the presence or absence of an analyte in a liquid sample, comprising:
(i) a sample receiving member;
(ii) a carrier in fluid communication with the sample receiving member;
(iii) a labeled reagent which is mobile in the carrier in the presence of the liquid sample, the labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, and wherein the coated nanoparticle does not include a Raman-active molecule; and
(iv) a binding reagent effective to capture the analyte, when present, immobilized in a defined detection zone of the carrier;
(b) contacting the liquid sample with the sample receiving member of the test device;
(c) allowing the liquid sample applied to the sample receiving member to mobilize said labeled reagent such that the liquid sample and labeled reagent move along the length of the carrier to pass into the detection zone;
(d) detecting the presence of the labeled reagent in the detection zone by measuring reflectance, wherein detection of the labeled reagent in the detection zone is indicative of the presence of analyte in the liquid sample, and failure to detect the presence of the labeled reagent in the detection zone is indicative of the absence of the analyte in the liquid sample.

2. The method of claim 1, wherein
(i) the carrier comprises nitrocellulose, plastic, or glass, preferably the carrier comprises nitrocellulose; or
(ii) the analyte is a protein, nucleic acid, metabolite, small molecule, virus, or bacterium; or
(iii) the test device further comprises an absorbent pad in fluid communication with the detection zone; or
(iv) the test device further comprises a control zone in fluid communication with the detection zone; or
(v) the presence of the analyte in the liquid sample is determined quantitatively; or
(vi) the test device is configured to detect multiple analytes, preferably the test device further comprises at least two different labeled reagents, wherein the ligands of the labeled reagents bind to different analytes, and at least two detection zones for detecting each of the at least two different labeled reagents.

3. A method for determining the presence or absence of an analyte in a liquid sample, comprising:
a) providing a test device comprising: (i) a sample receiving member; (ii) a carrier in fluid communication with the sample receiving member; and (iii) a binding reagent effective to capture the analyte, when present, immobilized in a defined detection zone of the carrier;
b) mixing the liquid sample with a labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, wherein the coated nanoparticle does not include a Raman-active molecule;
c) contacting the mixture of b) with the sample receiving member of the test device;
d) allowing the mixture of b) applied to the sample receiving member to move along the length of the carrier to pass into the detection zone;
e) detecting the presence of the labeled reagent in the detection zone by measuring reflectance, wherein detection of the labeled reagent in the detection zone is indicative of the presence of analyte in the liquid sample, and failure to detect the presence of the labeled reagent in the detection zone is indicative of the absence of the analyte in the liquid sample.

4. The method of claim 1 or 3, wherein
(i) a reflectance reader is used to detect the labeled reagent in the detection zone; or
(ii) the presence of analyte in the liquid sample is determined quantitatively; or
(iii) the method detects multiple analytes in the liquid sample, preferably the test device further comprises at least two different labeled reagents, wherein the ligands of the labeled reagents bind to different analytes, and at least two detection zones for detecting each of the at least two different labeled reagents; or
(iv) the metal cores of the nanoparticle are gold cores.

5. A method for determining the presence or absence of an analyte in a liquid sample using a kit for performing a flow-through analytical test for detecting the presence or absence of an analyte in a liquid sample by reflectometry, said kit comprising:
(i) a test device comprising a porous membrane comprising an upper surface and a lower surface and a binding reagent effective to capture the analyte, when present in the liquid sample, attached to the upper or lower surface of the porous membrane; and
(ii) a labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, wherein the coated nanoparticle does not include a Raman-active molecule,
said method comprising:
(a) contacting the liquid sample with the upper surface of the porous membrane; (b) allowing the liquid sample to flow through the porous membrane such that at least a portion of the analyte, when present in the liquid sample, binds to the binding reagent;
(c) contacting the labeled reagent with the upper surface of the porous membrane;
(d) allowing the labeled reagent to flow through the porous membrane such that at least a portion of the labeled reagent binds to the analyte; and
(e) detecting the presence of the labeled reagent on the porous membrane by measuring reflectance, wherein detection of the labeled reagent on the porous membrane is indicative of the presence of the analyte in the liquid sample, and failure to detect the presence of the labeled reagent on the porous membrane is indicative of the absence of the analyte in the liquid sample.

6. A method for determining the presence or absence of an analyte in a liquid sample using a kit for performing a flow-through analytical test for detecting the presence or absence of an analyte in a liquid sample by reflectometry, said kit comprising:
(i) a test device comprising a porous membrane comprising an upper surface and a lower surface and a binding reagent effective to capture the analyte, when present in the liquid sample, attached to the upper or lower surface of the porous membrane; and
(ii) a labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, wherein the coated nanoparticle does not include a Raman-active molecule,
said method comprising:
(a) mixing the liquid sample with the labeled reagent such that the analyte, when present in the liquid sample, binds to the labeled reagent;
(b) contacting the mixture of (a) with the upper surface of the porous membrane;
(c) allowing the mixture of (a) to flow through the porous membrane such that at least a portion of the analyte bound to the labeled reagent binds to the binding reagent; and
(d) detecting the presence of the labeled reagent on the porous membrane by measuring reflectance, wherein detection of the labeled reagent on the porous membrane is indicative of the presence of analyte in the liquid sample, and failure to detect the presence of the labeled reagent on the porous membrane is indicative of the absence of the analyte in the liquid sample.

7. The method of claim 5 or 6, wherein
(i) a reflectance reader is used to detect the labeled reagent on the porous membrane; or
(ii) the presence of analyte in the liquid sample is determined quantitatively; or
(iii) the method detects multiple analytes in the liquid sample, preferably the test device further comprises at least two different labeled reagents, wherein the ligands of the labeled reagents bind to different analytes, and at least two detection zones for detecting each of the at least two different labeled reagents; or
(iv) the metal cores of the nanoparticle are gold cores.

8. The method of claim 5 or 6, wherein in the kit:
(i) the analyte is a protein, nucleic acid, metabolite, small molecule, virus, or bacterium; or
(ii) the test device further comprises an absorbent pad, wherein the lower surface of the porous membrane and the absorbent pad are in physical contact and in fluid communication, and wherein the binding reagent is attached to the upper surface of the porous membrane; or
(iii) the test device further comprises a housing for the porous membrane; or
(iv) the presence of analyte in the liquid sample is determined quantitatively; or
(v) the test device is configured to detect multiple analytes, preferably the test device further comprises at least two different labeled reagents, wherein the ligands of the labeled reagents bind to different analytes, and at least two detection zones for detecting each of the at least two different labeled reagents.

9. A system comprising
(I) a test device for determining the presence or absence of an analyte in a liquid sample, comprising:
(i) a sample receiving member;
(ii) a carrier in fluid communication with the sample receiving member;
(iii) a labeled reagent which is mobile in the carrier in the presence of the liquid sample, the labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, and wherein the coated nanoparticle does not include a Raman-active molecule; and
(iv) a binding reagent effective to capture the analyte, when present, immobilized in a defined detection zone of the carrier; wherein the liquid sample applied to the sample receiving member mobilizes the labeled reagent such that the sample and labeled reagent are transported along the length of the carrier to pass into the detection zone, and wherein detection of the labeled reagent in the detection zone is indicative of the presence of analyte in the liquid sample, and
(II) a reflectometer adapted to detect the presence of the labeled reagent in the test device.

10. The system of claim 9, wherein in the test device
(i) the carrier comprises nitrocellulose, plastic, or glass, preferably the carrier comprises nitrocellulose; or
(ii) the analyte is a protein, nucleic acid, metabolite, small molecule, virus, or bacterium; or
(iii) the test device further comprises an absorbent pad in fluid communication with the detection zone; or
(iv) the test device further comprises a control zone in fluid communication with the detection zone; or
(v) the presence of the analyte in the liquid sample is determined quantitatively; or
(vi) the test device is configured to detect multiple analytes, preferably the test device further comprises at least two different labeled reagents, wherein the ligands of the labeled reagents bind to different analytes, and at least two detection zones for detecting each of the at least two different labeled reagents; or
(vii) the metal cores of the nanoparticle are gold cores..

11. A system comprising
(I) a a kit for performing a flow-through analytical test for detecting the presence or absence of an analyte in a liquid sample by reflectometry, comprising:
(i) a test device comprising a porous membrane comprising an upper surface and a lower surface and a binding reagent effective to capture the analyte, when present in the liquid sample, attached to the upper or lower surface of the porous membrane; and
(ii) a labeled reagent comprising a ligand that binds to the analyte and a coated nanoparticle consisting of multiple metal cores and one shell having the ligand attached thereto, wherein the shell comprises glass, a ceramic material such as perovskite and ZrO₂, or a polymer such as polyethylene glycol, polymethylmethacrylate and polystyrene, and increases the reflectance of the nanoparticle, wherein the coated nanoparticle does not include a Raman-active molecule, and
(II) a reflectometer adapted to detect the presence of the labeled reagent in the test device.

12. The system of claim 11, wherein in the kit
(i) the analyte is a protein, nucleic acid, metabolite, small molecule, virus, or bacterium; or
(ii) the test device further comprises an absorbent pad, wherein the lower surface of the porous membrane and the absorbent pad are in physical contact and in fluid communication, and wherein the binding reagent is attached to the upper surface of the porous membrane; or
(iii) the test device further comprises a housing for the porous membrane; or
(iv) the presence of analyte in the liquid sample is determined quantitatively; or
(v) the test device is configured to detect multiple analytes, preferably the test device further comprises at least two different labeled reagents, wherein the ligands of the labeled reagents bind to different analytes, and at least two detection zones for detecting each of the at least two different labeled reagents; or
(vii) the metal cores of the nanoparticle are gold cores.

## Patentansprüche

1. Verfahren zur Bestimmung der An- oder Abwesenheit eines Analyten in einer flüssigen Probe, umfassend:
(a) Bereitstellen einer Testvorrichtung zur Bestimmung der An- oder Abwesenheit eines Analyten in einer flüssigen Probe, umfassend:
(i) ein Probenaufnahmeelement;
(ii) einen Träger in Fluidkommunikation mit dem Probenaufnahmeelement;
(iii) ein markiertes Reagens, das in dem Träger in Anwesenheit der flüssigen Probe mobil ist, wobei das markierte Reagens einen Liganden, der an den Analyten bindet, und ein beschichtetes Nanoteilchen, das aus mehreren Metallkernen und einer Hülle, an die der Ligand gebunden ist, besteht, umfasst, wobei die Hülle Glas, ein Keramikmaterial, wie Perowskit und ZrO₂, oder ein Polymer, wie Polyethylenglycol, Polymethylmethacrylat und Polystyrol, umfasst und das Reflexionsvermögen des Nanoteilchens erhöht und wobei das beschichtete Nanoteilchen kein Raman-aktives Molekül enthält; und
(iv) ein Bindungsreagens, das ein Abfangen des Analyten, wenn einer vorhanden ist, bewirkt und in einer definierten Nachweiszone des Trägers immobilisiert ist;
(b) In-Kontakt-Bringen der flüssigen Probe mit dem Probenaufnahmeelement der Testvorrichtung;
(c) Zulassen, dass die auf das Probenaufnahmeelement aufgetragene flüssige Probe das markierte Reagens mobilisiert, so dass sich die flüssige Probe und das markierte Reagens entlang der Länge des Trägers bewegen und in die Nachweiszone eintreten;
(d) Nachweisen der Anwesenheit des markierten Reagens in der Nachweiszone durch Messen des Reflexionsvermögens, wobei der Nachweis des markierten Reagens in der Nachweiszone auf die Anwesenheit des Analyten in der flüssigen Probe hindeutet und das Nichtnachweisen der Anwesenheit des markierten Reagens in der Nachweiszone auf die Abwesenheit des Analyten in der flüssigen Probe hindeutet.

2. Verfahren gemäß Anspruch 1, wobei
(i) der Träger Nitrocellulose, Kunststoff oder Glas umfasst und vorzugsweise der Träger Nitrocellulose umfasst; oder
(ii) der Analyt ein Protein, eine Nucleinsäure, ein Metabolit, ein kleines Molekül, ein Virus oder Bakterium ist; oder
(iii) die Testvorrichtung weiterhin ein saugfähiges Feld in Fluidkommunikation mit der Nachweiszone umfasst; oder
(iv) die Testvorrichtung weiterhin eine Kontrollzone in Fluidkommunikation mit der Nachweiszone umfasst; oder
(v) die Anwesenheit des Analyten in der flüssigen Probe quantitativ bestimmt wird; oder
(vi) die Testvorrichtung so konfiguriert ist, dass sie mehrere Analyten nachweisen kann, wobei vorzugsweise die Testvorrichtung weiterhin wenigstens zwei verschiedene markierte Reagentien, wobei die Liganden der markierten Reagentien an unterschiedliche Analyten binden, und wenigstens zwei Nachweiszonen zum jeweiligen Nachweis der wenigstens zwei verschiedenen markierten Reagentien umfasst.

3. Verfahren zur Bestimmung der An- oder Abwesenheit eines Analyten in einer flüssigen Probe, umfassend:
a) Bereitstellen einer Testvorrichtung, die (i) ein Probenaufnahmeelement, (ii) einen Träger in Fluidkommunikation mit dem Probenaufnahmeelement und (iii) ein Bindungsreagens, das ein Abfangen des Analyten, wenn einer vorhanden ist, bewirkt und in einer definierten Nachweiszone des Trägers immobilisiert ist, umfasst;
b) Mischen der flüssigen Probe mit einem markierten Reagens, das einen Liganden, der an den Analyten bindet, und ein beschichtetes Nanoteilchen, das aus mehreren Metallkernen und einer Hülle, an die der Ligand gebunden ist, besteht, umfasst, wobei die Hülle Glas, ein Keramikmaterial, wie Perowskit und ZrO₂ oder ein Polymer, wie Polyethylenglycol, Polymethylmethacrylat und Polystyrol, umfasst und das Reflexionsvermögen des Nanoteilchens erhöht, wobei das beschichtete Nanoteilchen kein Raman-aktives Molekül enthält;
c) In-Kontakt-Bringen des Gemischs von b) mit dem Probenaufnahmeelement der Testvorrichtung;
d) Zulassen, dass sich das auf das Probenaufnahmeelement aufgetragene Gemisch von b) entlang der Länge des Trägers bewegt und in die Nachweiszone eintritt;
e) Nachweisen der Anwesenheit des markierten Reagens in der Nachweiszone durch Messen des Reflexionsvermögens, wobei der Nachweis des markierten Reagens in der Nachweiszone auf die Anwesenheit des Analyten in der flüssigen Probe hindeutet und das Nichtnachweisen der Anwesenheit des markierten Reagens in der Nachweiszone auf die Abwesenheit des Analyten in der flüssigen Probe hindeutet.

4. Verfahren gemäß Anspruch 1 oder 3, wobei
(i) ein Reflexionsvermögen-Lesegerät verwendet wird, um das markierte Reagens in der Nachweiszone nachzuweisen; oder
(ii) die Anwesenheit des Analyten in der flüssigen Probe quantitativ bestimmt wird; oder
(iii) das Verfahren mehrere Analyten in der flüssigen Probe nachweist, wobei vorzugsweise die Testvorrichtung weiterhin wenigstens zwei verschiedene markierte Reagentien, wobei die Liganden der markierten Reagentien an unterschiedliche Analyten binden, und wenigstens zwei Nachweiszonen zum jeweiligen Nachweis der wenigstens zwei verschiedenen markierten Reagentien umfasst; oder
(iv) die Metallkerne des Nanoteilchens Goldkerne sind.

5. Verfahren zur Bestimmung der An- oder Abwesenheit eines Analyten in einer flüssigen Probe unter Verwendung eines Kits zur Durchführung eines analytischen Durchflusstests zum Nachweisen der An- oder Abwesenheit eines Analyten in einer flüssigen Probe durch Reflektometrie, wobei der Kit umfasst:
(i) eine Testvorrichtung, die eine poröse Membran mit einer oberen Fläche und einer unteren Fläche sowie ein Bindungsreagens, das ein Abfangen des Analyten, wenn einer vorhanden ist, bewirkt und an der oberen oder unteren Fläche der porösen Membran befestigt ist, umfasst; und
(ii) ein markiertes Reagens, das einen Liganden, der an den Analyten bindet, und ein beschichtetes Nanoteilchen, das aus mehreren Metallkernen und einer Hülle, an die der Ligand gebunden ist, besteht, umfasst, wobei die Hülle Glas, ein Keramikmaterial, wie Perowskit und ZrO₂, oder ein Polymer, wie Polyethylenglycol, Polymethylmethacrylat und Polystyrol, umfasst und das Reflexionsvermögen des Nanoteilchens erhöht und wobei das beschichtete Nanoteilchen kein Raman-aktives Molekül enthält;
wobei das Verfahren umfasst:
(a) In-Kontakt-Bringen der flüssigen Probe mit der oberen Fläche der porösen Membran;
(b) Fließenlassen der flüssigen Probe durch die poröse Membran, so dass wenigstens ein Teil des Analyten, wenn einer in der flüssigen Probe vorhanden ist, an das Bindungsreagens bindet;
(c) In-Kontakt-Bringen des markierten Reagens mit der oberen Fläche der porösen Membran;
(d) Fließenlassen des markierten Reagens durch die poröse Membran, so dass wenigstens ein Teil des markierten Reagens an den Analyten bindet; und
(e) Nachweisen der Anwesenheit des markierten Reagens auf der porösen Membran durch Messen des Reflexionsvermögens, wobei der Nachweis des markierten Reagens auf der porösen Membran auf die Anwesenheit des Analyten in der flüssigen Probe hindeutet und das Nichtnachweisen der Anwesenheit des markierten Reagens auf der porösen Membran auf die Abwesenheit des Analyten in der flüssigen Probe hindeutet.

6. Verfahren zur Bestimmung der An- oder Abwesenheit eines Analyten in einer flüssigen Probe unter Verwendung eines Kits zur Durchführung eines analytischen Durchflusstests zum Nachweisen der An- oder Abwesenheit eines Analyten in einer flüssigen Probe durch Reflektometrie, wobei der Kit umfasst:
(i) eine Testvorrichtung, die eine poröse Membran mit einer oberen Fläche und einer unteren Fläche sowie ein Bindungsreagens, das ein Abfangen des Analyten, wenn einer in der flüssigen Probe vorhanden ist, bewirkt und an der oberen oder unteren Fläche der porösen Membran befestigt ist, umfasst; und
(ii) ein markiertes Reagens, das einen Liganden, der an den Analyten bindet, und ein beschichtetes Nanoteilchen, das aus mehreren Metallkernen und einer Hülle, an die der Ligand gebunden ist, besteht, umfasst, wobei die Hülle Glas, ein Keramikmaterial, wie Perowskit und ZrO₂, oder ein Polymer, wie Polyethylenglycol, Polymethylmethacrylat und Polystyrol, umfasst und das Reflexionsvermögen des Nanoteilchens erhöht und wobei das beschichtete Nanoteilchen kein Raman-aktives Molekül enthält;
wobei das Verfahren umfasst:
(a) Mischen der flüssigen Probe mit dem markierten Reagens, so dass der Analyt, wenn einer in der flüssigen Probe vorhanden ist, an das markierte Reagens bindet;
(b) In-Kontakt-Bringen des Gemischs von (a) mit der oberen Fläche der porösen Membran;
(c) Fließenlassen des Gemischs von (a) durch die poröse Membran, so dass wenigstens ein Teil des Analyten, der an das markierte Reagens gebunden ist, an das Bindungsreagens bindet; und
(d) Nachweisen der Anwesenheit des markierten Reagens auf der porösen Membran durch Messen des Reflexionsvermögens, wobei der Nachweis des markierten Reagens auf der porösen Membran auf die Anwesenheit des Analyten in der flüssigen Probe hindeutet und das Nichtnachweisen der Anwesenheit des markierten Reagens auf der porösen Membran auf die Abwesenheit des Analyten in der flüssigen Probe hindeutet.

7. Verfahren gemäß Anspruch 5 oder 6, wobei
(i) ein Reflexionsvermögen-Lesegerät verwendet wird, um das markierte Reagens auf der porösen Membran nachzuweisen; oder
(ii) die Anwesenheit des Analyten in der flüssigen Probe quantitativ bestimmt wird; oder
(iii) das Verfahren mehrere Analyten in der flüssigen Probe nachweist, wobei vorzugsweise die Testvorrichtung weiterhin wenigstens zwei verschiedene markierte Reagentien, wobei die Liganden der markierten Reagentien an unterschiedliche Analyten binden, und wenigstens zwei Nachweiszonen zum jeweiligen Nachweis der wenigstens zwei verschiedenen markierten Reagentien umfasst; oder
(iv) die Metallkerne des Nanoteilchens Goldkerne sind.

8. Verfahren gemäß Anspruch 5 oder 6, wobei in dem Kit
(i) der Analyt ein Protein, eine Nucleinsäure, ein Metabolit, ein kleines Molekül, ein Virus oder Bakterium ist; oder
(ii) die Testvorrichtung weiterhin ein saugfähiges Feld umfasst, wobei die untere Fläche der porösen Membran und das saugfähige Feld miteinander in physikalischem Kontakt und in Fluidkommunikation sind und wobei das Bindungsreagens an der oberen Fläche der porösen Membran befestigt ist; oder
(iii) die Testvorrichtung weiterhin ein Gehäuse für die poröse Membran umfasst; oder
(iv) die Anwesenheit des Analyten in der flüssigen Probe quantitativ bestimmt wird; oder
(v) die Testvorrichtung so konfiguriert ist, dass sie mehrere Analyten nachweisen kann, wobei vorzugsweise die Testvorrichtung weiterhin wenigstens zwei verschiedene markierte Reagentien, wobei die Liganden der markierten Reagentien an unterschiedliche Analyten binden, und wenigstens zwei Nachweiszonen zum jeweiligen Nachweis der wenigstens zwei verschiedenen markierten Reagentien umfasst.

9. System, umfassend
(I) eine Testvorrichtung zur Bestimmung der An- oder Abwesenheit eines Analyten in einer flüssigen Probe, umfassend:
(i) ein Probenaufnahmeelement;
(ii) einen Träger in Fluidkommunikation mit dem Probenaufnahmeelement;
(iii) ein markiertes Reagens, das in dem Träger in Anwesenheit der flüssigen Probe mobil ist, wobei das markierte Reagens einen Liganden, der an den Analyten bindet, und ein beschichtetes Nanoteilchen, das aus mehreren Metallkernen und einer Hülle, an die der Ligand gebunden ist, besteht, umfasst, wobei die Hülle Glas, ein Keramikmaterial, wie Perowskit und ZrO₂, oder ein Polymer, wie Polyethylenglycol, Polymethylmethacrylat und Polystyrol, umfasst und das Reflexionsvermögen des Nanoteilchens erhöht und wobei das beschichtete Nanoteilchen kein Raman-aktives Molekül enthält; und
(iv) ein Bindungsreagens, das ein Abfangen des Analyten, wenn einer vorhanden ist, bewirkt und in einer definierten Nachweiszone des Trägers immobilisiert ist; wobei die auf das Probenaufnahmeelement aufgetragene flüssige Probe das markierte Reagens so mobilisiert, dass die Probe und das markierte Reagens entlang der Länge des Trägers transportiert werden und somit in die Nachweiszone eintreten, und wobei der Nachweis des markierten Reagens in der Nachweiszone auf die Anwesenheit des Analyten in der flüssigen Probe hindeutet; und
(II) ein Reflektometer, das geeignet ist, die Anwesenheit des markierten Reagens in der Testvorrichtung nachzuweisen.

10. System gemäß Anspruch 9, wobei in der Testvorrichtung
(i) der Träger Nitrocellulose, Kunststoff oder Glas umfasst und vorzugsweise der Träger Nitrocellulose umfasst; oder
(ii) der Analyt ein Protein, eine Nucleinsäure, ein Metabolit, ein kleines Molekül, ein Virus oder Bakterium ist; oder
(iii) die Testvorrichtung weiterhin ein saugfähiges Feld in Fluidkommunikation mit der Nachweiszone umfasst; oder
(iv) die Testvorrichtung weiterhin eine Kontrollzone in Fluidkommunikation mit der Nachweiszone umfasst; oder
(v) die Anwesenheit des Analyten in der flüssigen Probe quantitativ bestimmt wird; oder
(vi) die Testvorrichtung so konfiguriert ist, dass sie mehrere Analyten nachweisen kann, wobei vorzugsweise die Testvorrichtung weiterhin wenigstens zwei verschiedene markierte Reagentien, wobei die Liganden der markierten Reagentien an unterschiedliche Analyten binden, und wenigstens zwei Nachweiszonen zum jeweiligen Nachweis der wenigstens zwei verschiedenen markierten Reagentien umfasst; oder
(vii) die Metallkerne des Nanoteilchens Goldkerne sind.

11. System, umfassend
(I) einen Kit zur Durchführung eines analytischen Durchflusstests zum Nachweisen der An- oder Abwesenheit eines Analyten in einer flüssigen Probe durch Reflektometrie, umfassend:
(i) eine Testvorrichtung, die eine poröse Membran mit einer oberen Fläche und einer unteren Fläche sowie ein Bindungsreagens, das ein Abfangen des Analyten, wenn einer vorhanden ist, bewirkt und an der oberen oder unteren Fläche der porösen Membran befestigt ist, umfasst; und
(ii) ein markiertes Reagens, das einen Liganden, der an den Analyten bindet, und ein beschichtetes Nanoteilchen, das aus mehreren Metallkernen und einer Hülle, an die der Ligand gebunden ist, besteht, umfasst, wobei die Hülle Glas, ein Keramikmaterial, wie Perowskit und ZrO₂, oder ein Polymer, wie Polyethylenglycol, Polymethylmethacrylat und Polystyrol, umfasst und das Reflexionsvermögen des Nanoteilchens erhöht und wobei das beschichtete Nanoteilchen kein Raman-aktives Molekül enthält; und
(II) ein Reflektometer, das geeignet ist, die Anwesenheit des markierten Reagens in der Testvorrichtung nachzuweisen.

12. System gemäß Anspruch 11, wobei in dem Kit
(i) der Analyt ein Protein, eine Nucleinsäure, ein Metabolit, ein kleines Molekül, ein Virus oder Bakterium ist; oder
(ii) die Testvorrichtung weiterhin ein saugfähiges Feld umfasst, wobei die untere Fläche der porösen Membran und das saugfähige Feld miteinander in physikalischem Kontakt und in Fluidkommunikation sind und wobei das Bindungsreagens an der oberen Fläche der porösen Membran befestigt ist; oder
(iii) die Testvorrichtung weiterhin ein Gehäuse für die poröse Membran umfasst; oder
(iv) die Anwesenheit des Analyten in der flüssigen Probe quantitativ bestimmt wird; oder
(v) die Testvorrichtung so konfiguriert ist, dass sie mehrere Analyten nachweisen kann, wobei vorzugsweise die Testvorrichtung weiterhin wenigstens zwei verschiedene markierte Reagentien, wobei die Liganden der markierten Reagentien an unterschiedliche Analyten binden, und wenigstens zwei Nachweiszonen zum jeweiligen Nachweis der wenigstens zwei verschiedenen markierten Reagentien umfasst; oder
(vii) die Metallkerne des Nanoteilchens Goldkerne sind.

## Revendications

1. Procédé pour déterminer la présence ou l'absence d'un analyte dans un échantillon liquide, comprenant les étapes consistant à:
(a) fournir un dispositif d'essai permettant de déterminer la présence ou l'absence d'un analyte dans un échantillon liquide, comprenant :
(i) un élément conçu pour recevoir les échantillons ;
(ii) un support en communication fluidique avec l'élément conçu pour recevoir les échantillons ;
(iii) un réactif marqué qui est mobile dans le support en présence de l'échantillon liquide, ledit réactif marqué comprenant un ligand qui se lie à l'analyte et une nanoparticule enrobée constituée de noyaux métalliques multiples et d'une enveloppe sur laquelle se fixe le ligand, où l'enveloppe comprend du verre, un matériau céramique tel que la pérovskite et ZrO₂, ou un polymère tel que le polyéthylèneglycol, le poly(méthacrylate de méthyle) et le polystyrène, et augmente la réflectance de la nanoparticule et, où la nanoparticule enrobée ne comporte pas de molécule active à effet Raman ; et
(iv) un réactif de liaison efficace pour capturer, lorsqu'il est présent, l'analyte immobilisé dans une zone de détection définie du support ;
(b) mettre l'échantillon liquide en contact avec l'élément du dispositif d'essai, conçu pour recevoir les échantillons ;
(c) laisser l'échantillon liquide appliqué à l'élément conçu pour recevoir les échantillons mobiliser ledit réactif marqué de sorte que l'échantillon liquide et le réactif marqué se déplacent le long du support pour passer dans la zone de détection ;
(d) détecter la présence du réactif marqué dans la zone de détection en mesurant la réflectance, où la détection du réactif marqué dans la zone de détection est révélatrice de la présence d'analyte dans l'échantillon liquide, et la non détection de la présence du réactif marqué dans la zone de détection est révélatrice de l'absence de l'analyte dans l'échantillon liquide.

2. Procédé selon la revendication 1, dans lequel
(i) le support comprend de la nitrocellulose, du plastique ou du verre, de préférence, le support comprend de la nitrocellulose ; ou
(ii) l'analyte est une protéine, un acide nucléique, un métabolite, une petite molécule, un virus ou une bactérie ; ou
(iii) le dispositif d'essai comprend en outre un tampon absorbant en communication fluidique avec la zone de détection ; ou
(iv) le dispositif d'essai comprend en outre une zone de contrôle en communication fluidique avec la zone de détection ; ou
(v) la présence de l'analyte dans l'échantillon liquide est déterminée de manière quantitative ; ou
(vi) le dispositif d'essai est configuré pour détecter des analytes multiples, de préférence, le dispositif d'essai comprend en outre au moins deux réactifs marqués différents, où les ligands des réactifs marqués se lient à des analytes différents, et au moins deux zones de détection pour détecter chacun desdits au moins deux réactifs marqués différents.

3. Procédé pour déterminer la présence ou l'absence d'un analyte dans un échantillon liquide, comprenant les étapes consistant à:
a) fournir un dispositif d'essai comprenant : (i) un élément conçu pour recevoir les échantillons ; (ii) un support en communication fluidique avec l'élément conçu pour recevoir les échantillons ; et (iii) un réactif de liaison efficace pour capturer, lorsqu'il est présent, l'analyte immobilisé dans une zone de détection définie du support ;
b) mélanger l'échantillon liquide avec un réactif marqué comprenant un ligand qui se lie à l'analyte et une nanoparticule enrobée constituée de noyaux métalliques multiples et d'une enveloppe sur laquelle se fixe le ligand, où l'enveloppe comprend du verre, un matériau céramique tel que la pérovskite et ZrO₂, ou un polymère tel que le polyéthylèneglycol, le poly(méthacrylate de méthyle) et le polystyrène, et augmente la réflectance de la nanoparticule, où la nanoparticule enrobée ne comporte pas de molécule active à effet Raman ;
c) mettre en contact le mélange de b) avec l'élément du dispositif d'essai, conçu pour recevoir les échantillons ;
d) laisser le mélange de b) appliqué à l'élément conçu pour recevoir les échantillons se déplacer le long du support pour passer dans la zone de détection ;
e) détecter la présence du réactif marqué dans la zone de détection en mesurant la réflectance, où la détection du réactif marqué dans la zone de détection est révélatrice de la présence d'analyte dans l'échantillon liquide, et la non détection de la présence du réactif marqué dans la zone de détection est révélatrice de l'absence de l'analyte dans l'échantillon liquide.

4. Procédé selon la revendication 1 ou 3, où
(i) un lecteur de réflectance est utilisé pour détecter le réactif marqué dans la zone de détection ; ou
(ii) la présence d'analyte dans l'échantillon liquide est déterminée de manière quantitative ; ou
(iii) ledit procédé détecte des analytes multiples dans l'échantillon liquide, de préférence, le dispositif d'essai comprend en outre au moins deux réactifs marqués différents, où les ligands des réactifs marqués se lient à des analytes différents, et au moins deux zones de détection pour détecter chacun desdits au moins deux réactifs marqués différents ; ou
(iv) les noyaux métalliques de la nanoparticule sont des noyaux d'or.

5. Procédé pour déterminer la présence ou l'absence d'un analyte dans un échantillon liquide en utilisant une trousse pour réaliser un essai analytique à flux continu pour détecter la présence ou l'absence d'un analyte dans un échantillon liquide par réflectométrie, ladite trousse comprenant :
(i) un dispositif d'essai comprenant une membrane poreuse comportant une surface supérieure et une surface inférieure et un réactif de liaison efficace pour capturer, lorsqu'il est présent dans l'échantillon liquide, l'analyte fixé sur la surface supérieure ou la surface inférieure de la membrane poreuse; et
(ii) un réactif marqué comprenant un ligand qui se lie à l'analyte et une nanoparticule enrobée constituée de noyaux métalliques multiples et d'une enveloppe sur laquelle se fixe le ligand, où l'enveloppe comprend du verre, un matériau céramique tel que la pérovskite et ZrO₂, ou un polymère tel que le polyéthylèneglycol, le poly(méthacrylate de méthyle) et le polystyrène, et augmente la réflectance de la nanoparticule, où la nanoparticule enrobée ne comporte pas de molécule active à effet Raman,
ledit procédé comprenant les étapes consistant à:
(a) mettre l'échantillon liquide en contact avec la surface supérieure de la membrane poreuse ; (b) laisser l'échantillon liquide s'écouler à travers la membrane poreuse de sorte qu'au moins une portion de l'analyte, lorsqu'il est présent dans l'échantillon liquide, se lie au réactif de liaison ;
(c) mettre le réactif marqué en contact avec la surface supérieure de la membrane poreuse ;
(d) laisser le réactif marqué s'écouler à travers la membrane poreuse de sorte qu'au moins une portion du réactif marqué se lie à l'analyte ; et
(e) détecter la présence du réactif marqué sur la membrane poreuse en mesurant la réflectance, où la détection du réactif marqué sur la membrane poreuse est révélatrice de la présence de l'analyte dans l'échantillon liquide, et la non détection de la présence du réactif marqué sur la membrane poreuse est révélatrice de l'absence de l'analyte dans l'échantillon liquide.

6. Procédé pour déterminer la présence ou l'absence d'un analyte dans un échantillon liquide en utilisant une trousse pour réaliser un essai analytique à flux continu pour détecter la présence ou l'absence d'un analyte dans un échantillon liquide par réflectométrie, ladite trousse comprenant :
(i) un dispositif d'essai comprenant une membrane poreuse comportant une surface supérieure et une surface inférieure et un réactif de liaison efficace pour capturer, lorsqu'il est présent dans l'échantillon liquide, l'analyte fixé sur la surface supérieure ou la surface inférieure de la membrane poreuse; et
(ii) un réactif marqué comprenant un ligand qui se lie à l'analyte et une nanoparticule enrobée constituée de noyaux métalliques multiples et d'une enveloppe sur laquelle se fixe le ligand, où l'enveloppe comprend du verre, un matériau céramique tel que la pérovskite et ZrO₂, ou un polymère tel que le polyéthylèneglycol, le poly(méthacrylate de méthyle) et le polystyrène, et augmente la réflectance de la nanoparticule, où la nanoparticule enrobée ne comporte pas de molécule active à effet Raman,
ledit procédé comprenant les étapes consistant à:
(a) mélanger l'échantillon liquide avec le réactif marqué de sorte que, lorsqu'il est présent dans l'échantillon liquide, l'analyte se lie au réactif marqué ;
(b) mettre le mélange de (a) en contact avec la surface supérieure de la membrane poreuse ;
(c) laisser le mélange de (a) s'écouler à travers la membrane poreuse de sorte qu'au moins une portion de l'analyte lié au réactif marqué se lie au réactif de liaison; et
(d) détecter la présence du réactif marqué sur la membrane poreuse en mesurant la réflectance, où la détection du réactif marqué sur la membrane poreuse est révélatrice de la présence de l'analyte dans l'échantillon liquide, et la non détection de la présence du réactif marqué sur la membrane poreuse est révélatrice de l'absence de l'analyte dans l'échantillon liquide.

7. Procédé selon la revendication 5 ou 6, où
(i) un lecteur de réflectance est utilisé pour détecter le réactif marqué sur la membrane poreuse ; ou
(ii) la présence d'analyte dans l'échantillon liquide est déterminée de manière quantitative ; ou
(iii) ledit procédé détecte des analytes multiples dans l'échantillon liquide, de préférence, le dispositif d'essai comprend en outre au moins deux réactifs marqués différents, où les ligands des réactifs marqués se lient à des analytes différents, et au moins deux zones de détection pour détecter chacun desdits au moins deux réactifs marqués différents ; ou
(iv) les noyaux métalliques de la nanoparticule sont des noyaux d'or.

8. Procédé selon la revendication 5 ou 6 où, dans la trousse :
(i) l'analyte est une protéine, un acide nucléique, un métabolite, une petite molécule, un virus ou une bactérie ; ou
(ii) le dispositif d'essai comprend en outre un tampon absorbant, où la surface inférieure de la membrane poreuse et le tampon absorbant sont physiquement en contact et en communication fluidique, et où le réactif de liaison est fixé sur la surface supérieure de la membrane poreuse; ou
(iii) le dispositif d'essai comprend en outre un boîtier pour la membrane poreuse ; ou
(iv) la présence d'analyte dans l'échantillon liquide est déterminée de manière quantitative ; ou
(v) le dispositif d'essai est configuré pour détecter des analytes multiples, de préférence, le dispositif d'essai comprend en outre au moins deux réactifs marqués différents, où les ligands des réactifs marqués se lient à des analytes différents, et au moins deux zones de détection pour détecter chacun desdits au moins deux réactifs marqués différents.

9. Système comprenant
(I) un dispositif d'essai permettant de déterminer la présence ou l'absence d'un analyte dans un échantillon liquide, comprenant :
(i) un élément conçu pour recevoir les échantillons ;
(ii) un support en communication fluidique avec l'élément conçu pour recevoir les échantillons ;
(iii) un réactif marqué qui est mobile dans le support en présence de l'échantillon liquide, ledit réactif marqué comprenant un ligand qui se lie à l'analyte et une nanoparticule enrobée constituée de noyaux métalliques multiples et d'une enveloppe sur laquelle se fixe le ligand, où l'enveloppe comprend du verre, un matériau céramique tel que la pérovskite et ZrO₂, ou un polymère tel que le polyéthylèneglycol, le poly(méthacrylate de méthyle) et le polystyrène, et augmente la réflectance de la nanoparticule, et où la nanoparticule enrobée ne comporte pas de molécule active à effet Raman ; et
(iv) un réactif de liaison efficace pour capturer, lorsqu'il est présent, l'analyte immobilisé dans une zone de détection définie du support ; où l'échantillon liquide appliqué à l'élément conçu pour recevoir les échantillons mobilise le réactif marqué de sorte que l'échantillon et le réactif marqué sont transportés le long du support pour passer dans la zone de détection ; et où la détection du réactif marqué dans la zone de détection est révélatrice de la présence de l'analyte dans l'échantillon liquide, et
(II) un réflectomètre adapté pour détecter la présence du réactif marqué dans le dispositif d'essai.

10. Système selon la revendication 9 où, dans le dispositif d'essai
(i) le support comprend de la nitrocellulose, du plastique ou du verre, de préférence, le support comprend de la nitrocellulose ; ou
(ii) l'analyte est une protéine, un acide nucléique, un métabolite, une petite molécule, un virus ou une bactérie ; ou
(iii) le dispositif d'essai comprend en outre un tampon absorbant en communication fluidique avec la zone de détection ; ou
(iv) le dispositif d'essai comprend en outre une zone de contrôle en communication fluidique avec la zone de détection ; ou
(v) la présence de l'analyte dans l'échantillon liquide est déterminée de manière quantitative ; ou
(vi) le dispositif d'essai est configuré pour détecter des analytes multiples, de préférence, le dispositif d'essai comprend en outre au moins deux réactifs marqués différents, où les ligands des réactifs marqués se lient à des analytes différents, et au moins deux zones de détection pour détecter chacun desdits au moins deux réactifs marqués différents ; ou
(vii) les noyaux métalliques de la nanoparticule sont des noyaux d'or.

11. Système comprenant
(I) une trousse pour réaliser un essai analytique à flux continu pour détecter la présence ou l'absence d'un analyte dans un échantillon liquide par réflectométrie, comprenant :
(i) un dispositif d'essai comprenant une membrane poreuse comportant une surface supérieure et une surface inférieure et un réactif de liaison efficace pour capturer, lorsqu'il est présent dans l'échantillon liquide, l'analyte fixé sur la surface supérieure ou la surface inférieure de la membrane poreuse; et
(ii) un réactif marqué comprenant un ligand qui se lie à l'analyte et une nanoparticule enrobée constituée de noyaux métalliques multiples et d'une enveloppe sur laquelle se fixe le ligand, où l'enveloppe comprend du verre, un matériau céramique tel que la pérovskite et ZrO₂, ou un polymère tel que le polyéthylèneglycol, le poly(méthacrylate de méthyle) et le polystyrène, et augmente la réflectance de la nanoparticule, où la nanoparticule enrobée ne comporte pas de molécule active à effet Raman, et
(II) un réflectomètre adapté pour détecter la présence du réactif marqué dans le dispositif d'essai.

12. Système selon la revendication 11 où, dans la trousse
(i) l'analyte est une protéine, un acide nucléique, un métabolite, une petite molécule, un virus ou une bactérie ; ou
(ii) le dispositif d'essai comprend en outre un tampon absorbant, où la surface inférieure de la membrane poreuse et le tampon absorbant sont physiquement en contact et en communication fluidique, et où le réactif de liaison est fixé sur la surface supérieure de la membrane poreuse; ou
(iii) le dispositif d'essai comprend en outre un boîtier pour la membrane poreuse ; ou
(iv) la présence d'analyte dans l'échantillon liquide est déterminée de manière quantitative ; ou
(v) le dispositif d'essai est configuré pour détecter des analytes multiples, de préférence, le dispositif d'essai comprend en outre au moins deux réactifs marqués différents, où les ligands des réactifs marqués se lient à des analytes différents, et au moins deux zones de détection pour détecter chacun desdits au moins deux réactifs marqués différents; ou
(vii) les noyaux métalliques de la nanoparticule sont des noyaux d'or.
